Europäisches Patentamt

European Patent Office

Office européen des brevets

(19)

(11) **EP 0 670 181 B1**

(12) # EUROPÄISCHE PATENTSCHRIFT

(45) Veröffentlichungstag und Bekanntmachung des Hinweises auf die Patenterteilung:
**19.05.1999 Patentblatt 1999/20**

(51) Int. Cl.$^6$: **B01J 21/16**, B01J 29/06

(21) Anmeldenummer: **95102454.6**

(22) Anmeldetag: **21.02.1995**

(54) **Katalysator- bzw. Katalysatorträger-Formkörper**

Catalyst, in particular shaped supported catalyst

Catalyseur, en particulier catalyseur sur support mis en forme

(84) Benannte Vertragsstaaten:
**DE ES GB**

(30) Priorität: **23.02.1994 DE 4405877**

(43) Veröffentlichungstag der Anmeldung:
**06.09.1995 Patentblatt 1995/36**

(73) Patentinhaber: **SÜD-CHEMIE AG**
**D-80333 München (DE)**

(72) Erfinder:
- **Engelhardt, Thomas, Dr.**
  **D-85356 Freising (DE)**
- **Hähn, Reinhard, Dr.**
  **D-84186 Vilsheim (DE)**

(74) Vertreter: **Reitzner, Bruno, Dr.**
**Patentanwälte Dipl.-Ing. R. Splanemann**
**Dr. B. Reitzner, Dipl.-Ing. K. Baronetzky**
**Tal 13**
**80331 München (DE)**

(56) Entgegenhaltungen:
**EP-A- 0 313 167**      **DE-A- 3 738 916**
**GB-A-  884 633**      **US-A- 5 192 726**

- **ULLMANN: "Encyklopädie der technichen Chemie, 4. Ausgabe (1983), Band 23, S.322/3" , , VERLAG CHEMIE, WEINHEIM**

**Beschreibung**

Die Erfindung betrifft Katalysator- bzw. Katalysatorträger-Formkörper auf der Basis mindestens eines säureaktivierten Schichtsilicats. Derartige Katalysator- bzw. Katalysatorträger-Formkörper sind als Handelsprodukte bekannt. Hingewiesen wird beispielsweise auf den Prospekt "K-Catalysts" der Anmelderin vom Juli 1976 mit weiteren Literaturhinweisen. So können beispielsweise die Katalysatoren KSF, KP 10 und KSF O in Granulatfrom geliefert werden.

Katalysator-Formkörper haben gegenüber Katalysatoren in Pulverform den Vorteil, daß sie z. B. in Festbettreaktoren in kontinuierlichen Prozessen eingesetzt werden können, da sie den Vorteil der leichteren Aktivierbarkeit und/oder Regenerierbarkeit im Reaktor haben und gegenüber den Reaktanten einen geringeren Strömungswiderstand haben als eine Pulverschüttung.

Die bekannten Katalysatorträger-Formkörper wurden allgemein dadurch hergestellt, daß ein Schichtsilicat auf Montmorillonit-Basis in der Alkali- oder Erdalkaliform uner Zusatz von Wasser granuliert wurde und die Granulate durch Behandlung mit Säure in die säureaktivierte Form übergeführt wurden (Austausch der Alkali- und Erdalkaliionen durch Protonen und gegebenenfalls Herauslösen eines Teils der Aluminiumkomponente). Die Säureaktivierung erfolgte jedoch verhältnismäßig langsam und war wegen der Inhomogenität des Schichtsilicats technisch nur schwer beherrschbar. Außerdem ließ die katalytische Aktivität der aus den Formkörpern hergestellten Katalysatoren zu wünschen übrig.

Aus der EP-B-0 031 687 ist ein Verfahren zur Durchführung von protonen-katalysierten organischen Reaktionen bekannt, wobei als Protonenquelle ein wasserstoffionen-ausgetauschter Schichtton, wie Bentonit, verwendet wird, der frei von anhaftender Säure ist. Die Katalysatorteilchen können zu Formkörpern verpreßt sein. Diese haben aber nur eine geringe Porosität, so daß die Aktivität verhältnismäßig gering ist.

Aus der EP-B-0 313 905 ist ein Verfahren zur direkten Hydratation von Olefinen mit Methanol und einem tertiären Alkohol in Gegenwart eines sauren Katalysators bekannt. Als saurer Katalysator kann beispielsweise mit Schwefelsäure behandelter Bentonit verwendet werden, der aber nicht als Formkörper eingesetzt wird. Dieser Katalysator kann nicht für kontinuierliche Reaktionen verwendet werden.

Aus der DE-C-41 25 031 ist ein Verfahren zur Herstellung von hydroxylgruppenhaltigen Fettsäureverbindungen durch Umsetzung von epoxidierten Fettsäureverbindungen mit mittelständigen Oxirangruppen mit Wasser in Anwesenheit von säureaktivierten Tonerden und/oder Silicaten und/oder Aktivkohlen bekannt. Die verwendeten säureaktivierten Tonerden liegen nicht als Formkörper vor und enthalten keine mikroporöse Kieselsäure.

Aus der DE-A-35 29 060 ist ein Katalysator zur Verringerung des Stickoxidgehalts von Verbrennungabgasen bekannt, welcher mindestens eines der Metalle Ti, Zr, V, W oder Ce in Form eines oder mehrerer ihrer Oxide enthält. Die wirksame Komponente des Katalysators ist eine Kombination eines oder mehrerer dieser Oxide mit einem sauren Alumosilicat mit Schichtstruktur. Hierunter fallen beispielsweise auch säureaktivierte Schichtsilicate. Der Katalysator enhält jedoch keine mikroporöse Kieselsäuren und/oder Silicate.

Die GB-A-884 633 beschreibt einen Katalysator, der aus einem synthetischen Hydrogel (Silicahydrogel), Magnesiumoxid und einem kristallinen Tonmineral z.B. aus einem Mineral der Kaolinit-, Halloysit- oder Montmorillonit-Gruppe hergestellt wird. Säureaktivierte Tonminerale, die infolge der Entfernung der Aluminiumionen aus dem Gitter durch einen hohen Anteil an amorpher Kieselsäure, eine hohe spezifische Oberfläche und ein hohes Mikroporenvolumen gekennzeichnet sind, werden nicht verwendet.

Der Erfindung lag die Aufgabe zugrunde, Katalysator- bzw. Katalysatorträger-Formkörper auf der Basis mindestens eines säureaktivierten Schichtsilicats bereitzustellen, die auf einfache Weise in einer homogenen Zusammensetzung herstellbar sind, und die als Katalysatoren eine hohe katalytische Aktivität besitzen.

Diese Aufgabe wird erfindungsgemäß dadurch gelöst, daß die Katalysator- bzw. Katalysatorträger-Formkörper mikroporöse Kieselsäuren und/oder Silicate enthalten.

Das Ausgangs-Schichtsilicat kann ein natürliches oder synthetisches Dreischichtsilicat sein, das vorzugsweise aus der Gruppe der Smectite (wie Montmorillonit, Hectorit, Antigorit, Nontronit, Beidellit oder Saponit) ausgewählt ist.

Andere brauchbare Ausgangs-Schichtsilicate sind Sepiolith, Attapulgit (Palygorskit), Stevensit oder Chlorit, unter den montmorillonithaltigen Mineralen sind insbesondere Bentonit und Fuller's Earth zu nennen, die je nach Fundort unterschiedlich zusammengesetzt sein können.

Die mikroporösen Kieselsäuren/Silicate haben vorzugsweise ein Mikroporenvolumen (Porendurchmesser von 0 bis 80 nm) von 0,05 bis 2,0 ml/g, insbesondere von 0,1 bis 1,5 ml/g.

Das Mikroporenvolumen wird nach einer Methode bestimmt, die auf der Kapillarkondensation von Tetrachlorkohlenstoff in den Poren des porösen Zusatzmittels beruht. Nach dieser Methode werden die Poren mit einem Durchmesser bis zu 80 nm erfaßt. Das Prinzip der Methode ist bei Benesi, Bonnar und Lee "Anal. Chem." 27 (1955) S. 1963 ff. angegeben. Im einzelnen werden hierbei 1 bis 2 g des zu prüfenden Zusatzmittels in einem Wägegläschen bei 130 °C im Trockenschrank getrocknet. Nach dem Abkühlen in einem Exsiccator wird das genau abgewogene Gläschen in einen Vakuumexsiccator gestellt, der eine Mischung aus 26 ml Paraffinöl und 184 ml Tetrachlorkohlenstoff enthält.

Durch dieses Mischungsverhältnis wird der maximale Porendurchmesser festgelegt, bei dem noch eine Kondensation des Tetrachlorkohlenstoffs stattfindet. Der mit graduierter Kühlfalle, Manometer und Vakuumpumpe verbundene Exsiccator wird nun bis zum Sieden des Inhalts evakuiert. Es werden 10 ml Tetrachlorkohlenstoff verdampft und in der Kühlfalle aufgefangen. Im Exsiccator liegt dann eine reine Tetrachlokohenstoffatmosphäre vor, aus der der Tetrachlorkohlenstoff in den Poren des Zusatzmittels kondensiert wird. Sodann läßt man den Exsiccatorinhalt 16 bis 20 Stunden bei Raumtemperatur (22 °C) ausgleichen, wobei die Kondensation des Tetrachlorkohlenstoffs vervollständigt wird. Anschließend läßt man langsam Luft in den Exsiccator. Nach dem Abnehmen des Exsiccatordeckels wird das Wägegläschen sofort verschlossen auf der Analysenwaage zurückgewogen.

Auswertung:

$$\text{Porenvolumen (ml/g)} = \frac{\text{Einwaage - Auswaage}}{\text{Einwaage x Dichte CCl}_4 \ (1{,}595 \ \text{g/m}^3)}$$

Bei den mikroporösen Kieselsäuren bzw. Silicaten handelt es sich vorzugsweise um Kieselgele, Fällungskieselsäuren, Kieselgur und amorphe oder kristalline Aluminiumsilicate. Weniger bevorzugt werden basische mikroporöse Calcium- bzw. Magnesiumsilicate. Als mikroporöse Silicate sind auch die sogenannten "Pillared Clays" sehr gut geeignet.

Die mikroporösen Kieselsäuren bzw. Silicate sind üblicherweise in einer Menge von 5 bis 5 Gew.-% bzw. von 5 bis 20 Gew.-% vorhanden. Ihre Teilchengröße ist relativ unkritisch, sollte aber vorzugsweise unter 250 µm liegen. Besonders bevorzugt liegt die Teilchengröße zwischen etwa 5 und 200 µm.

Gegenstand der Erfindung ist ferner ein Verfahren zur Herstellung der vorstehend definierten Katalysator- bzw. Katalysatorträger-Formkörper, das dadurch gekennzeichnet ist, daß man (a) ein feinteiliges Schichtsilicat mit den mikroporösen Kieselsäuren/Silicaten vermischt, (b) die Mischung gegebenenfalls unter Zusatz eines wäßrigen Mediums zu Formkörpern formt, (c) die Formkörper gegebenenfalls trocknet, (d) das in den Formkörpern enthaltene Schichtsilicat durch Behandlung mit Säure aktiviert, (e) die bei der Säureaktivierung gebildeten Salze sowie die überschüssige Säure abtrennt und (f) die Formkörper trocknet.

Bei der Säureaktivierung wird das Schichtsilicat mit einer beliebigen anorganischen und/oder organischen Säure, vorzugsweise einer Mineralsäure, wie Salzsäure, Phosphorsäure oder Schwefelsäure, umgesetzt. Hierbei erfolgt eine Vergrößerung der spezifischen Oberfläche, was im allgemeinen eine Erhöhung der Aktivität zur Folge hat. Besonders bevorzugt wird Schwefelsäure bei Raumtemperatur verwendet. Es hat sich herausgestellt, daß die Reaktionszeiten durch den Zusatz der mikroporösen Kieselsäuren bzw. Silicate erheblich reduziert werden können. Auch die katalytische Aktivität der die mikroporösen Kieselsäuren bzw. Silicate enthaltenden Formkörper ist wesentlich höher als die Aktivität der säureaktivierten Schichtsilicat-Formkörper ohne diesen Zusatz.

Die erfindungsgemäßen Katalysator- bzw. Katalysatorträger-Formkörper können Granulate, Kugeln, Ringe, Tabletten, Zylinder, Speichenräder, Extrudate, 'fancy shapes' und Wabenkörper sein.

Gegenstand der Erfindung ist ferner die Verwendung der vorstehend definierten Katalysatoren für säurekatalysierte Reaktionen. Hierzu zählen beispielsweise Verätherungen, Veresterungen, Kondensationsreaktionen, Acetalisierungen, Hydratisierungen, Alkylierungen, die Acylierung von Aromaten, die Transalkylierung und Dealkylierung von Alkylaromaten, Isomerisierungen, aromatische Cyclisierungen, Dimerisierungen, Oligomerisierungen, Polymerisationen und Disproportionierungen.

Die erfindungsgemäßen Katalysator-Formkörper können beispielsweise für alle Reaktionen verwendet werden, wie sie in dem vorstehend genannten Prospekt "K-Catalysts" beschrieben sind.

Die beschriebenen Prozesse können in der Flüssigphase, Gasphase oder Trickelphase (Dreiphasenreaktoren) durchgeführt werden. Die Prozesse werden vorzugsweise kontinuierlich durchgeführt, können aber auch ansatzweise im Batchbetrieb durchgeführt werden. In den kontinuierlichen Prozessen werden die Zusammensetzung und die Konzentration der Reaktanten, der Druck, die Temperatur und die Raumgeschwindigkeit in geeigneter Weise variiert. In Batchprozessen werden die Zusammensetzung und die Konzentration der Reaktanten, der Druck, die Temperatur und die Verweilzeit in geeigneter Weise variiert.

Die Prozesse werden bevorzugt in Festbettreaktoren, gegebenenfalls in Reaktoren mit Rührwerk, durchgeführt.

Die Dreiphasenreaktoren werden im Gleichstrom oder im Gegenstrom betrieben.

Die Edukt- und/oder Produktströme können über die Katalysatorschüttung rezyklisiert und mit unter den Reaktionsbedingungen gasförmigen und/oder flüssigen Stoffen verdünnt werden. Als Verdünnungsmittel sind in der Regel Paraffine, Olefine, Naphthene und/oder Aromaten, Stickstoff, Wasserstoff und/oder Dampf geeignet.

Die Erfindung ist durch die nachstehenden Beispiele erläutert.

Vergleichsbeispiel

1.500 g vermahlener Calciumbentonit (Teilchengröße ≤ 100 μm) werden unter Zusatz von 450 ml Wasser im Eirich-Intensiv-Mischer zu einem Granulat verarbeitet. Das Wasser wird über einen Zeitraum von etwa 1/2 Minute zugesetzt. Die Mischzeit wird so eingestellt, daß vorzugsweise ein Granulat zwischen 1 und 5 mm anfällt. Das Granulat wird anschließend bei 150°C auf eine Restfeuchte von <5 Gew.-% getrocknet.

Das getrocknete Extrudat wird anschließend in eine Glasfilternutsche gegeben und bei Raumtemperatur mit 2000 ml konzentrierter Schwefelsäure bei Raumtemperatur überschichtet. Nach einer Reaktionszeit von 2 Stunden läßt man die Schwefelsäure ab und wäscht das Katalysatorgranulat mit Isopropanol. Anschließend wird das Granulat unter Vakuum bei Raumtemperatur getrocknet. Das Mikroporenvolumen des Granulats beträgt 0,04 ml/g.

Beispiel 1

Die Arbeitsweise von Vergleichsbeispiel 1 wird mit der Abweichung wiederholt, daß statt Calciumbentonit eine Mischung aus 1.200 g Calciumbentonit und 300 g Kieselgel (M 60 der Fa. Merck, Darmstadt; Mikroporenvolumen 0,6 ml/g; Teilchengröße 90 % < 100 μm) granuliert wird. Die Säureaktivierung mit Schwefelsäure erfolgt ebenfalls wie nach dem Vergleichsbeispiel.

Beispiel 2

1.200 g Calciumbetonit und 300 g Kieselgel von Beispiel 1 werden mit 500 ml Wasser im Eirich-Intensiv-Mischer plastifiziert und anschließend mit einem Einschneckenextruder zu 2 mm-Extrudaten verformt.

Die Extrudate werden anschließend bei 150°C auf eine Restfeuchte von 5 Gew.-% getrocknet und auf eine durchschnittliche Länge von 2 bis 5 mm gebrochen.

Die getrockneten Extrudate werden anschließend in eine Glasfilternutsche gegeben und mit 2000 ml konzentrierter Schwefelsäure bei Raumtemperatur überschichtet. Nach einer Reaktionszeit von 2 Stunden läßt man die Schwefelsäure ab und wäscht das Katalysatorgranulat mit Isopropanol. Anschließend wird das Granulat unter Vakuum bei Raumtemperatur getrocknet.

Beispiel 3

Die Arbeitsweise von Vergleichsbeispiel 1 wird mit der Abweichung wiederholt, daß 1.200 g Calciumbentonit und 300 g einer Fällungskieselsäure (Mikroporenvolumen 1,3 ml/g; Sipernat 50; Degussa, Frankfurt; Teilchengröße 90 % < 50 μm) im Eirich-Mischer granuliert werden. Die weitere Verarbeitung erfolgt wie nach dem Vergleichsbeispiel.

Beispiel 4

Die Arbeitsweise nach dem Vergleichsbeispiel wird mit der Abweichung wiederholt, daß 1.200 g Calciumbentonit mit 300 g Kieselgur (Mikroporenvolumen 0,35 ml/g; Celatom FN1 der Fa. Horn & Kappes) granuliert werden.

Beispiel 5

Die Arbeitsweise nach dem Vergleichsbeispiel wird mit der Abweichung wiederholt, daß 1.200 g Calciumbentonit mit 300 g Aluminiumsilicat (Mikroporenvolumen 0,8 ml/g; Teilchengröße 90 % < 40 μm; P 820; Degussa, Frankfurt) granuliert werden. Die weitere Behandlung erfolgt wie nach dem Vergleichsbeispiel.

Beispiel 6

Die Arbeitsweise nach dem Vergleichsbeispiel wird mit der Abweichung wiederholt, daß 1.300 g Calciumbentonit mit 300 g eines Zeolithen (Mikroporenvolumen 0,7 ml/g; Teilchengröße 90 % < 30 μm; Wessalith P, Degussa, Frankfurt) granuliert werden.

Die katalytische Aktivät der nach den Beispielen erhaltenen Formkörper (Veresterung von Essigsäure mit Ethanol zu Ethylacetat) wurde wie folgt bestimmt:

In einem 250-ml-Dreihalskolben, der mit einem Thermometer und mit einem Rückflußkühler ausgerüstet ist, werden

| | |
|---|---|
| 72 g | Essigsäure 100%ig (1,2 mol) mit |
| 55,2 g | Ethanol absolut (1,2 mol) |

gegeben und mit jeweils 10 g der nach den Beispielen erhaltenen Katalysatoren-Formkörper versetzt. Das Gemisch wird unter Rückfluß gekocht. Nach einer Reaktionszeit von 60 Minuten läßt man das Reaktionsgemisch abkühlen.

Aus dem erkalteten Reaktionsgemisch wird eine Probe von 1 g entnommen und in einem Erlenmeyer-Kolben gegeben, der 50 ml deionisiertes Wasser enthält. Der Gehalt an nicht umgesetzter Essigsäure wird durch Titration mit 1 n Natronlauge gegen Phenolphthalein bestimmt. Aus der Verhältnis der Essigsäurekonzentration vor und nach der Reaktion ergibt sich der Umsatz.

Die erhaltenen Ergebnisse sind in der nachstehenden Tabelle angegeben:

| Katalytische Aktivität | |
|---|---|
| Beispiel | Umsatz % (bezogen auf Essigsäure) nach 1 Stunde) |
| Vergleichsbeispiel | 25 |
| Beispiel 1 (Kieselgel) | 32 |
| Beispiel 2 (Kieselgel/Extrudat) | 35 |
| Beispiel 3 (Fällungskieselsäure) | 38 |
| Beispiel 4 (Kieselgur) | 29 |
| Beispiel 5 (amorphes Aluminiumsilicat) | 42 |
| Beispiel 6 (Zeolith) | 34 |

## Patentansprüche

1. Katalysator- bzw. Katalysatorträger-Formkörper auf der Basis mindestens eines säureaktivierten Schichtsilicats, gekennzeichnet durch einen Gehalt an mikroporösen Kieselsäuren und/oder Silicaten.

2. Formkörper nach Anspruch 1, dadurch gekennzeichnet, daß das Schichtsilicat aus der Gruppe der natürlichen oder synthetischen Dreischichtsilicate ausgewählt ist.

3. Formkörper nach einem der Ansprüche 1 oder 2, dadurch gekennzeichnet, daß die mikroporösen Kieselsäuren/Silicate ein Mikroporenvolumen (0 bis 80 nm) von etwa 0,05 ml/g bis 2,0 ml/g, vorzugsweise von etwa 0,1 bis 1,5ml/g haben.

4. Formkörper nach einem der Ansprüche 1 bis 3, dadurch gekennzeichnet, daß die mikroporösen Kieselsäuren/Silicate, Kieselgele, Fällungskieselsäuren, Kieselgur oder amorphe oder kristalline Aluminiumsilicate darstellen.

5. Formkörper nach einem der Ansprüche 1 bis 4, dadurch gekennzeichnet, daß die mikroporösen Kieselsäuren/Silicate in Mengen von etwa 5 bis 50 Gew.-%, vorzugsweise in Mengen von etwa 5 bis 20 Gew.-% vorhanden sind.

6. Formkörper nach einem der Ansprüche 1 bis 5, dadurch gekennzeichnet, daß die mikroporösen Kieselsäuren/Silicate eine Teilchengröße von < 250 µm, vorzugsweise von etwa 5 bis 200 µm haben.

7. Verfahren zur Herstellung der Katalysator- bzw. Katalysatorträger-Formkörper nach einem der Ansprüche 1 bis 6, dadurch gekennzeichnet, daß man (a) ein feinteiliges Schichtsilicat mit den mikroporösen Kieselsäuren/Silicaten vermischt; (b) die Mischung, gegebenenfalls unter Zusatz eines wäßrigen Mediums, zu Formkörpern formt; (c) die Formkörper gegebenenfalls trocknet; (d) das in den Formkörpern enthaltene Schichtsilicat durch Behandlung mit Säure aktiviert; (e) die bei der Säureaktivierung gebildeten Salze sowie die überschüssige Säure abtrennt; und (f) die Formkörper trocknet.

8. Verfahren nach Anspruch 7, dadurch gekennzeichnet, daß man zur Säureaktivierung des Schichtsilicats eine Mineralsäure, vorzugsweise Schwefelsäure, verwendet.

9. Verwendung der Formkörper nach einem der Ansprüche 1 bis 6 bzw. der nach den Ansprüchen 7 oder 8 hergestellten Formkörper für säurekatalysierte Reaktionen.

## Claims

1. Catalyst or catalyst carrier moldings on the basis of at least one acid-activated layer silicate, characterised by a content of microporous silicic acids and/or silicates

2. The moldings acording to Claim 1, characterized in that the layer silicate is selected from the group of natural or synthetic three-layer silicates.

3. The moldings according to any one of Claims 1 to 2, characterized in that the microporous silicic acids/silicates have a micropore volume (0 bis 80 nm) of about 0,05 ml/g to 2,0 ml/g, preferably of about 0,1 to 1,5 ml/g.

4. The moldings according to any one of Claims 1 to 3, characterized in that the microporous silicic acids/silicates are silica gels, precipitated silicas, kieselguhr or amorphous or crystalline aluminium silicates.

5. The moldings according to any one of Claims 1 to 4, characterized in that the microporous silicic acids/silicates are present in proportions of about 5 to 50 percent by weight, preferably in proportions of about 5 to 20 percent by weight.

6. The moldings according to any one of Claims 1 to 5, characterized in that the microporous silicic acids/silicates have a particle size < 250 μm, preferably of about 5 to 200 μm.

7. A process for producing the catalyst or catalyst carrier moldings according to any one of Claims 1 to 6, characterized by (a) mixing a finely divided layer silicate with the microporous silicic acids/silicates; (b) optionally shaping the mixture, by adding an aqueous medium, into moldings; (c) activating the layer silicate contained in the moldings by treatment with acid; (e) separating the salts produced in the acid activation, and the excess acid; and (f) drying the moldings.

8. The process according to Claim 7, characterized in that a mineral acid, preferably a sulfuric acid, is used for acid activation of the layer silicate.

9. The use of the moldings according to any one of Claims 1 to 6 or of the moldings produced according to Claims 7 or 8 for acid catalysed reactions.

## Revendications

1. Corps moulé de catalyseur ou de support de catalyseur à base d'au moins un silicate en feuillets activé à l'acide, caractérisé en ce qu'il contient des acides siliciques et/ou des silicates microporeux.

2. Corps moulé selon la revendication 1, caractérisé en ce que le silicate en feuillets est choisi dans le groupe des silicates naturels ou synthétiques ayant une structure à 3 feuillets.

3. Corps moulé de catalyseur ou de support de catalyseur selon l'une des revendications 1 ou 2, caractérisé en ce que les acides siliciques/silicates microporeux présentent un volume de micropores (0 à 80 nm) d'environ 0,05 à 2,0 ml/g, de préférence d'environ 0,1 à 1,5 ml/g.

4. Corps moulé selon l'une quelconque des revendications 1 à 3, caractérisé en ce que les acides siliciquès/silicates microporeux sont des gels de silice, de l'acide silicique obtenu par précipitation, du kieselguhr ou des silicates d'aluminium amorphes ou cristallins.

5. Corps moulé selon l'une quelconque des revendications 1 à 4, caractérisé en ce que les acides siliciques/silicates microporeux sont présents en des quantités d'environ 5 à 50% en poids, de préférence en des quantités d'environ 5 à 20% en poids.

6. Corps moulé selon l'une quelconque des revendications 1 à 5, caractérisé en ce que les acides siliciques/ silicates microporeux ont une granulométrie < 250 μm, de préférence de 5 à 200 μm.

7. Procédé de fabrication des corps moulés de catalyseur ou de support de catalyseur selon l'une quelconque des revendications 1 à 6, caractérisé en ce que : (a) un silicate à structure en feuillets finement divisé est mélangé avec les acides siliciques/silicates microporeux ; (b) les corps susmentionnés sont obtenus par formage dudit mélange, le cas échéant avec adjonction d'un milieu aqueux ; (c) les corps sont le cas échéant séchés ; (d) le silicate à structure en feuillets contenu dans ces corps est activé par traitement à l'acide ; (e) les sels formés lors de cette activation à l'acide ainsi que l'acide excédentaire sont éliminés ; (f) les corps sont séchés.

8. Procédé selon la revendication 7, caractérisé en ce que l'activation du silicate à structure en feuillets est réalisée au moyen d'un acide minéral, de préférence de l'acide sulfurique.

9. Utilisation des corps moulés selon l'une quelconque des revendications 1 à 6, ou encore des corps moulés fabriqués selon les revendications 7 ou 8, dans des réactions de catalyse acide.